(19) Europäisches Patentamt

European Patent Office

Office européen des brevets

(11) Publication number: **0 258 780 B1**

(12) **EUROPEAN PATENT SPECIFICATION**

(45) Date of publication of patent specification: **23.06.93**  (51) Int. Cl.5: **C08G 63/66**, C08G 63/64, A61K 9/22

(21) Application number: **87112279.2**

(22) Date of filing: **25.08.87**

(54) **Polyesters containing alkylene oxide blocks as drug delivery systems.**

(30) Priority: **05.09.86 US 903801**
**05.09.86 US 903797**

(43) Date of publication of application:
**09.03.88 Bulletin 88/10**

(45) Publication of the grant of the patent:
**23.06.93 Bulletin 93/25**

(84) Designated Contracting States:
**AT BE CH DE ES FR GB GR IT LI LU NL SE**

(56) References cited:
**EP-A- 0 098 394**
**EP-A- 0 108 933**
**DE-A- 2 206 144**

**Rev. Macromol. Chem. Phys., C23(1), 87-92 (1983).**

**Polymer, 20, 1459 (1979).**

(73) Proprietor: **AMERICAN CYANAMID COMPANY**
**1937 West Main Street P.O. Box 60**
**Stamford Connecticut 06904-0060(US)**

(72) Inventor: **Casey, Donald James**
**9 Revere Place**
**Ridgefield Connecticut 06877(US)**
Inventor: **Rosati, Louis**
**183 Sunrise Hill Road**
**Norwalk Connecticut 06851(US)**
Inventor: **Jarrett, Peter Kendrick**
**24 Chatfield Drive**
**Trumbull Connecticut 06611(US)**

(74) Representative: **Wächtershäuser, Günter, Dr.**
**Tal 29**
**W-8000 München 2 (DE)**

## Description

It is known from EP-108933 that triblock copolymers (ABA) having a middle block B being a poly-(alkylene oxide) and end blocks A consisting of glycolic acid ester linkages are biodegradable melt-moldable materials. The known materials are used for producing absorbable surgical articles and compositions for the controlled continuous administration of a pharmaceutical drug to a living animal.

EP-A-0098394 discloses surgical articles and drug delivery systems, manufactured from a glycolide-trimethylene carbonate triblock copolymer. Monofilament sutures prepared from this type of triblock copolymer exhibit improved flexibility and extended strength retention compared to known absorption sutures while retaining an acceptable absorption rate.

The present invention provides a slow release drug delivery system as defined in claim 1 comprising a drug, preferably bovine somatotropin (bST) and an ABA block polymer wherein the (B) block is a poly-(ethylene oxide) and the blocks (A) are comprised of degradable random copolymers of (1) glycolide and (2) trimethylene carbonate.

The poly(ethylene oxide) concentration in the block polymer is within the range of about 4 to about 54 weight percent of the block polymer and preferably about 4 to about 30 weight percent and the ratio of glycolide to trimethylene carbonate is within a range of about 45 weight percent glycolide and about 55 weight percent trimethylene carbonate to about 68 weight percent glycolide and about 32 weight percent trimethylene carbonate. The average molecular weight of the B component, poly(ethylene oxide), is with in a range of about 5000 to about 20,000.

In the triblock copolymer, the middle block is obtained by removing both terminal hydroxyl hydrogens from a homopolymer of ethylene oxide.

Each end block of the triblock copolymer consists essentially of glycolic acid esters and trimethylene carbonate linkages. In a specific embodiment, the middle block is from 5 to 25 percent by weight of the copolymer. In a more specific embodiment, the number average molecular weight of the middle block is from about 4,000 to 30,000.

In a most specific embodiment (to the above specific embodiments), a bioabsorbable triblock copolymer has been invented. The inherent viscosity of the copolymer, as measured at 30°C for a 0.5% (w/v) solution in chloroform or methylene chloride, is 0.25 to about 1.50 dl/g.

In an aqueous environment, the thermoplastic hydrogels consisting of ABA block polymers will swell to a predetermined equilibrium value and will release a wide variety of high molecular weight (>1000) biologically active materials. In addition, the materials are capable of being completely degraded and eliminated from the body over a period of time. A particular advantage of these materials is their thermoplastic nature; that is, they can be processed by conventional solution or thermal techniques.

Recently, there has been interest in using hydrogels in a wide variety of biomedical (including veterinary) applications such as contact lenses, burn dressings, blood and tissue compatible implants, and drug delivery devices. In the area of controlled drug delivery devices, cross-linked hydrogel materials have met with great success. However, these materials suffer drawbacks, such as a lack of processibility, which are a consequence of their cross-linked nature.

Our approach to this problem was to investigate the use of ABA block copolymers as thermoplastic degradable hydrogels. In these block polymers, the (B) block is a water soluble polymer such as poly-(ethylene oxide) and the blocks (A) are comprised of degradable random copolymers of glycolide (Gly) and trimethylene carbonate (TMC). The middle and end blocks of the block copolymer are chemically incompatible and the result is a phase separated system with crystalline alkylene oxide regions dispersed throughout the Gly/TMC matrix. When exposed to an aqueous environment, the block copolymer segments pick up an amount of water which is a function of the composition and molecular weight of the various block structures. In addition, the low glass transition temperature of the random Gly/TMC blocks allows for facile deformation of the matrix to occur on swelling. This is necessary to accommodate the dimensional changes brought about by the swelling process. Typically, the polyethylene oxides used as B blocks include hydroxyl ended polyethylene oxide.

Slow release drug delivery systems of the invention may be used as implants or parenteral suspensions prepared from pharmaceutically and pharmacologically acceptable liquid vehicle.

### Polymerization Method

The method of choice for preparing the above block copolymers including the ABA triblock copolymers is the melt phase ring-opening copolymerization of glycolide and trimethylene carbonate using specially purified, commercially available difunctional poly(ethylene glycols) as initiators. These polymerizations are conducted in a stirred reactor at 165°C under nitrogen. When maximum melt viscosity has been reached, the polymer is discharged and allowed to cool to room temperature. The polymers can be purified by reprecipitation for methylene chloride solutions into methanol or ethanol.

Determination of Water Uptake

Samples of the above polymers are extruded at 60°-100°C on an extruder to yield fibers of 1,5 mm average diameter. The fibers are then cut into ~2.54 cm (~1") lengths and several are placed in deionized water at room temperature. At various time intervals, the fibers are withdrawn, wiped thoroughly to remove any surface liquid, and the water uptake is measured gravimetrically. Alternatively, the uptake can be measured with thin films (0.6 mm) prepared by compression molding the polymer at 90°C, or by casting thin films of the polymer from solution.

Fabrication Methods

A. Solution Casting

A solution of polymer (20-50% w/v) is prepared in an appropriate low boiling solvent such as methylene chloride. A biologically active material that is insoluble in methylene chloride, such as bovine somatotropin (bST), is added with rapid stirring to form a viscous slurry. The proportions are chosen so that the active material is 1-75% of the weight of the final dry device. The slurry is then poured into a mold which has been pre-cooled to -78°C. After approximately 15 minutes, the frozen slab is placed in a freezer for 3-4 days to allow most of the solvent to evaporate. Final drying of the solution cast disk is accomplished in a vacuum oven at room temperature. The disk can be cut into squares or, in the preferred method, cryogenically ground through a 841 $\mu$m (20 mesh) screen to give particles which are capable of being injected or implanted.

B. Coextrusion

The above polymers and a biologically active material are coextruded at 60-115°C on a laboratory scale extruder. The ratio of active material is chosen to be 1-50% w/w but is preferably 25-50% w/w. The 1.5 mm diameter fibers can be cut into lengths or cryogenically ground through a 841 $\mu$m (20 mesh) screen to give particles which are capable of being injected, or the fiber can be directly implanted.

In Vitro Release Measurements

A sample (0.5-2.5 g) of polymer which had been loaded with a biologically active material such as bST is placed into a polypropylene dissolution tube. To simulate physiological conditions, 30 ml of phosphate buffered saline at pH = 7.4 is added and the tube is capped. The dissolution tube is then rotated at 3-7 rpm in a water bath at 37°C. Periodically, an aliquot of solution is removed and replaced by fresh buffered saline. The aliquot is then analyzed for total protein content by using a biuret assay. The protein copper complex is measured spectrophotometrically at 540 nm and is compared to a calibration curve constructed with known amounts of an identical protein. In the preferred method, the entire buffer solution is decanted daily from the dissolution tube and replaced by 30 ml of fresh buffer solution. An aliquot of the decanted buffer solution is then analyzed by the biuret assay method as above.

In Vivo Release Measurements

Polymer which contains bST is ground through a 20 mesh screen and suspended in soybean oil. Six hypophysectomized (hypox) rats are injected with the polymer containing the bST. The amount injected is adjusted so that each animal receives 800 ug of bST. In addition, there are two control groups of six hypox rats. The first group (positive control) each receives 80 ug of bST in buffer daily for 10 days (800 ug total). The second control group receives daily injections of aqueous buffer (negative control). The average weight gains of the 3 groups are then measured over a 10-day period.

The above embodiments are more fully described in the following examples.

## Example 1

### Purification of Materials

DL-lactide: DL-lactide was purchased from Purac, Inc. One kilogram of DL-lactide is refluxed for 1 1/2 hours with toluene (1500 g) which has been dried by distillation from benzophenone ketyl. The residual water is removed from the DL-lactide by collection of the toluene/water azeotrope in a Dean-Stark trap. The dry DL-lactide solution is allowed to cool to room temperature and placed in the refrigerator overnight. The crystallized DL-lactide is then quickly filtered and dried in a vacuum oven at room temperature. Recrystallization yield is 84%.

Polyethylene Glycol-8,000: Polyethylene glycol-8,000 (PEG 8,000) (160 g) is dissolved in methanol (1600ml). The PEG solution is then freed of catalyst impurities and deionized by slowly passing the solution through a methanol conditioned indicating mixed bed anionic and cationic ion-exchange resin (Amberlite MB-3, Rohm and Haas Company, PA, U.S.A.). After elution from the column, the PEG is crystallized by placing the solution in a freezer overnight. The crystalline PEG is then filtered and air dried for 2 hours. The PEG is further purified by recrystallization from acetone (1600 ml). The recrystallized PEG is filtered and dried in a vacuum oven at room temperature overnight. Prior to polymerization, the desired amount of purified PEG is dried further by heating in a vacuum oven at $70°C$ with $P_2O_5$ as a desiccant. PEG-14,000 and PEG-20,000 are purified in the same way.

Pluronic F68: Pluronic F68 was purified by the same technique as described for PEG above but without the acetone recrystallization step. The methanol recrystallized Pluronic F68 was filtered and dried in a vacuum oven at room temperature. Prior to polymerization, the Pluronic F68 was further dried by heating in a vacuum oven at $70°C$ with $P_2O_5$ as a desiccant.

Pluronic P105: Pluronic P105 was purified by the same method described for PEG above. The polymer was recovered from the methanol solution using a rotary evaporator. Residual methanol was removed by drying in vacuum to constant weight. The material was not recrystallized from acetone. Prior to polymerization the Pluronic P105 was dried further by heating in a vacuum oven at $50°C$ with $P_2O_5$ as a desiccant.

Polyethylene Glycol Methyl Ether: Polyethylene glycol methyl ether, nominal molecular weight 5000, was purified in the same way as described for PEG above.

## Example 2

### Synthesis of (Gly/TMC)-(PEO 14,000)-(Gly/TMC) ABA Triblock Copolymer (Gly/PEO/TMC: 34/41/25)

A 250 ml flask is charged with PEG-14000 (50 g, 0.0036 mole). The flask is placed in a vacuum oven and the PEG is dried overnight under vacuum at $70°C$ with $P_2O_5$ as a drying agent. The flask is then placed in a glove bag under $N_2$. Glycolide (25.0 g, 0.21 mole) and trimethylene carbonate (25.0 g, 0.24 mole) are charged to the flask and the contents are melted and mixed under $N_2$. The monomer mixture is then quickly transferred into a stirred reactor which has been heated under a nitrogen flow at $165°C$. Stannous octoate (0.16 ml, $4.9 \times 10^{-4}$ mole) is then quickly charged to the reactor with the use of a syringe. The polymer melt is stirred at 40 rpm for approximately 3 hours at $165°C$. This time period corresponds to a maximum in the melt viscosity. The polymer is discharged from the reactor and allowed to cool to room temperature. A portion of the crude polymer (42.8 g) is dissolved in $CH_2Cl_2$ (250 ml) and reprecipitated dropwise into rapidly stirred absolute ethanol (3000 ml). After filtration and drying to constant weight, the reprecipitation yield was determined to be 96%. The inherent viscosity of the polymer (0.5%, in $CHCl_3$ at $30°C$ was 0.38 dL/g. The composition was analyzed by $^1$H-NMR and was found to be 34/41/25 weight percent Gly/PEO/TMC. The Tg of the polymer was $11°C$, the melting point (Tm) was $59°C$.

### Examples 3-14

Several polymers were prepared as in Example 2 with varying PEG contents and PEG molecular weights (Table I). In many of the Gly/PEO/TMC triblock copolymers, the charged ratio of Gly/TMC is 60/40 weight percent. This allows for maximum Tg of the rubbery end blocks ($9°C$) while still maintaining solubility in common organic solvents. Differential scanning calorimetry (DSC) clearly shows phase separation in these materials. The Tg of the rubbery end blocks ($7°-16°C$) is very close to the Tg of a 60/40 random Gly/TMC polymer. In addition, the Tm of the crystalline PEO segments are only lowered $5°-10°C$.

4

Example 15

Synthesis of (Gly/TMC)-(PEO-8000)-(Gly/TMC) ABA, (Gly/PEO/TMC) 59/6/35

Glycolide (117.0 g, 1.01 mole), trimethylene carbonate (71.0 g, 0.70 mole), PEG-8000 (12.0 g) and stannous octoate (0.33 ml 1.0 x $10^{-3}$ mole) were combined in a stirred reactor as in Example 2. The reaction mixture was then stirred at 169°C and 36-40 rpm for 1.5 hours. The polymer was recovered as in Example 2. The properties of this polymer are summarized in Table I.

Example 16

Synthesis of (Gly/TMC)-(PEO-8000)-Gly/TMC) ABA (Gly/PEO/TMC: 54/8/38)

Glycolide (110.4 g, 0.95 moles), trimethylene carbonate (73.6 g, 0.72 moles), PEG-8000 (16.0 g) and stannous octoate (0.32 ml, 9.96 x $10^{-4}$ moles) were combined and allowed to polymerize as in Example 15. The properties of this polymer as summarized in Table I.

Example 17

Synthesis of (Gly/TMC)-(PEO-8000)-Gly/TMC) ABA, (Gly/PEO/TMC: 54/10/36)

Glycolide (108.0 g, 0.93 moles), trimethylene carbonate (72.0 g, 0.71 moles), PEG-8000 (20.0 g) and stannous octoate (0.32 ml, 9.96 x $10^{-4}$ moles) were combined and allowed to polymerize as in Example 15. The properties of this material are summarized in Table I.

## TABLE I

### Glycolide/PEO/TMC Polymers

| Ex. | Charged Composition (Gly/PEO/TMC Wgt. %) | PEG MW | $\eta_{inh}$ (Solvent) | | Gly/PEO/TMC Composition by $^1$H-NMR(wt %) | | Tg (°C) | Tm (°C) |
|---|---|---|---|---|---|---|---|---|
| | | | As Polymerized | Reprecipitated | As Polymerized | Reprecipitated | | |
| 3 | 25/50/25 | 14,000 | --- | 0.40 ($CHCl_3$) | --- | 30/43/27 | -- | -- |
| 4 | 32/50/18 | 14,000 | --- | 0.45 ($CH_2Cl_2$) | --- | 31/54/15 | -- | -- |
| 5 | 48/20/32 | 14,000 | --- | 0.45 ($CHCl_3$) | --- | 49/19/32 | 16 | 57 |
| 6 | 54/10/36 | 14,000 | --- | 0.34 ($CH_2Cl_2$) | --- | 55/11/34 | 12 | 54 |
| 7 | 42/30/28 | 14,000 | 0.45 ($CH_2Cl_2$) | 0.45 ($CH_2Cl_2$) | --- | 44/29/27 | 15 | 58 |
| 8 | 42/30/28 | 8,000 | 0.40 ($CH_2Cl_2$) | 0.38 ($CH_2Cl_2$) | --- | 43/31/26 | 16 | 55 |
| 9 | 48/20/32 | 8,000 | 0.42 ($CH_2Cl_2$) | --- | 48/21/31 | --- | 14 | 55 |
| 10 | 54/10/36 | 8,000 | 0.46 ($CH_2Cl_2$) | 0.33 ($CHCl_3$) | 50/10/40 | 50/8/42 | 10 | 53 |
| 11 | 54/10/36 | 20,000 | --- | --- | --- | --- | 7 | 47 |
| 12 | 48/20/32 | 20,000 | --- | --- | --- | --- | 6 | 52 |

EP 0 258 780 B1

TABLE I (Continued)

## Glycolide/PEO/TMC Polymers

| Ex. | Charged Composition (Gly/PEO/TMC Wgt. %) | PEG MW | $\eta$inh (Solvent) As Polymerized | Reprecipitated | Gly/PEO/TMC Composition by $^1$H-NMR(wt %) As Polymerized | Reprecipitated | Tg (°C) | Tm (°C) |
|---|---|---|---|---|---|---|---|---|
| 13 | 42/30/28 | 20,000 | --- | --- | --- | --- | 11 | 54 |
| 14 | 57/5/38 | 8,000 | 0.41 (CHCl$_3$) | 0.38 (CHCl$_3$) | 57/5/38 | 58/5/37 | -- | -- |
| 15 | 58/6/36 | 8,000 | 0.42 (CHCl$_3$) | 0.40 (CHCl$_3$) | 59/6/35 | 59/6/35 | -- | -- |
| 16 | 55/8/37 | 8,000 | 0.44 (CHCl$_3$) | 0.42 (CHCl$_3$) | 53/8/39 | 54/8/38 | -- | -- |
| 17 | 54/10/36 | 8,000 | 0.45 (CHCl$_3$) | 0.40 (CHCl$_3$) | 54/10/36 | 54/10/36 | -- | -- |

EP 0 258 780 B1

Example 18 (Comparative)

Synthesis of (Gly/dl-Lact)-(PEO-8000)-(Gly/dl-Lact)  ABA, (Gly/dl-Lact/PEO: 36/54/10)

Glycolide (54.0 g, 0.46 moles), dl-lactide (81.0 g, 0.56 moles), PEG-8000 (15.0 g) and stannous octoate (0.32ml, 9.96 x 10$^{-4}$ moles) were combined and allowed to polymerize as in Example 2. The properties of this polymer are summarized in Table II.

Example 19 (Comparative)

Synthesis of (Gly/l-Lact)-(PEO-8000)-(Gly/l-Lact)  ABA: (Gly/l-Lact/PEO: 27/65/8)

Glycolide (53.2 g, 0.46 moles), l-lactide (130.8 g, 0.91 moles), PEG-8000 (16.0 g) and stannous octoate (0.05 ml, 1.56 x 10$^{-4}$ moles) are combined and allowed to polymerize by the procedure described in Example 15. The properties of this polymer are summarized in Table II.

Example 20 (Comparative)

Synthesis of (l-Lact/TMC)-(PEO-8000)-(l-Lact/TMC)  ABA, (l-Lact/TMC/PEO: 43/49/8)

l-Lactide (88.0 g, 0.61 moles), trimethylene carbonate (96.0 g, 0.94 moles), PEG-8000 (16.0 g) and stannous octoate (0.31 ml, 9.74 x 10$^{-4}$ moles) are combined and allowed to polymerize by the procedure described in Example 15. The properties of this polymer are summarized in Table II.

Example 21 (Comparative)

Synthesis of (Gly/dl-Lact)-(PEO-20,000)-(Gly/dl-Lact) ABA, (Gly/dl-Lact/PEO: 21/25/54)

dl-lactide (25.0 g, 0.17 moles), glycolide (25.0 g, 0.21 moles), PEG 20,000 (50.0 g) and stannous octoate (0.16 ml, 4.94 x 10$^{-4}$ moles) are combined and allowed to polymerize by the procedure described in Example 2. The properties of this polymer are described in Table II.

8

EP 0 258 780 B1

## Table II

## Terpolymers With PEO Midblocks and Various Endblocks

| | Ex. | Charged Composition | PEG MW | ηInh (Solvent) | | Composition by $^1$H-NMR(Wt%) | | Tg | Tm |
|---|---|---|---|---|---|---|---|---|---|
| | | | | As Polymerized | Reprecipitated | As Polymerized | Reprecipitated | | |
| (Comp.) | 18 | Gly/dl-lactide/ PEO: 36/54/10 | 8,000 | 0.49(CHCl$_3$) | 0.35(CHCl$_3$) | 36/54/10 | 36/54/10 | -- | -- |
| (Comp.) | 19 | Gly/l-lactide/ PEO: 27/65/8 | 8,000 | 0.73(CHCl$_3$) | ----- | 27/65/8 | ----- | 36 | -- |
| (Comp.) | 20 | l-Lactide/TMC/ PEO: 44/48/8 | 8,000 | 0.56(CHCl$_3$) | ----- | 43/49/8 | ----- | 0 | -- |
| (Comp.) | 21 | Gly/dl-lactide/ PEO: 25/25/50 | 20,000 | ----- | 0.43(CHCl$_3$) | ----- | 21/25/54 | 42 | 57 |

Example 22

Swelling Behavior of Examples 3, 4 and 21

A film was prepared by solution casting a 20% w/v solution of the polymer of Example 3 in $CH_2Cl_2$. After the solvent had evaporated overnight, the film was dried further under vacuum at room temperature overnight. Films made from the polymers of Example 3, 4 and 21 were placed in water at 37°C with stirring. After 24 hours, films from Example 3 and Example 4 had formed emulsions. By day 3, the film from Example 21 had also formed an emulsion.

Example 23

Swelling Behavior of Example 7  (Gly/PEO/TMC: 44/29/27)

A sample of the polymer from Example 7 (1.5 g) was extruded at 110°C on an extruder to yield a 1.5 mm diameter fiber. From the fiber 5 samples, lengths each approximately 2.54 cm (1") were cut. The samples were placed in deionized water at room temperature. Periodically, the samples were withdrawn, wiped dry, and the water uptake measured gravimetrically. The water uptake is shown in Table III. From the values at 1280 min., the equilibrium water uptake for fibers was calculated to be 232 ± 3%.

The same type of water uptake analysis was performed on 4 samples of films of the polymer of Example 7 (12 x 4 x 0.6 mm). The results are shown in Table III. The shorter time to reach an equilibrium value of water uptake in the films is attributable to the greater surface-to-volume ratio in the films.

## Table III

## Water Uptake by Fibers and Films of 44/29/27 Gly/PEO/TMC (Ex. 7)

| Fibers | | Films | |
|---|---|---|---|
| Time (min) | % $H_2O^A$ Uptake | Time (min) | % $H_2O^A$ Uptake |
| 5 | 31.1 | 5 | 136.7 |
| 18 | 60.9 | 22 | 238.7 |
| 32 | 89.3 | 35 | 271.0 |
| 45 | 107.9 | 63 | 279.5 |
| 65 | 133.6 | 81 | 282.2 |
| 90 | 158.2 | 216 | 279.1 |
| 118 | 183.7 | 363 | 253.5 |
| 148 | 204.3 | 1560 | 266.3 |
| 179 | 223.3 | | |
| 1155 | 237.6 | | |
| 1280 | 235.5 | | |

$$A = \frac{(Wt\ Swollen - Wt\ Dry)}{Wt\ Dry} \times 100$$

Example 24

Swelling of Various Hydrogels

Water uptake experiments were carried out on fibers of several Gly/PEO/TMC hydrogels and one Gly/dl-Lactide/PEO hydrogel (Table IV). Measurements were carried out at room temperature in deionized water. All reported equilibrium uptake values are averages of 4 or 5 samples.

11

## TABLE IV

### Combined Swelling Data on Polymers

| Example | Polymer | PEG MW | PEO Content (Wgt. %) | % H$_2$O Uptake | Teq |
|---|---|---|---|---|---|
| 14 | Gly/PEO/TMC | 8,000 | 5 | 27.9 ± 5.4[1,3] | 13 days |
| 10 | Gly/PEO/TMC | 8,000 | 8 | 124.1 ± 7.4[1,3] | 1 day |
| 10 | Gly/PEO/TMC | 8,000 | 10 | 11.3 ± 0.9[1,2] | 4 |
| 18 (Comp.)(ref.) | Gly/dl-lactide/PEO | 8,000 | 10 | 9.9 ± 1.3[1,3,5] | 5 |
| 9 | Gly/PEO/TMC | 8,000 | 21 | 163.0 ± 1.8[1,2] | 4 |
| 8 | Gly/PEO/TMC | 8,000 | 31 | 224.5 ± 15.1[1,2] | 4 |
| 6 | Gly/PEO/TMC | 14,000 | 11 | 125.8 ± 4.5[1,3] | 4 |
| 5 | Gly/PEO/TMC | 14,000 | 19 | 164.9 ± 11.2[1,3] | 4 |
| 7 | Gly/PEO/TMC | 14,000 | 29 | 235.9 ± 3.1[1,3] | 17 hrs. |
| 7 | Gly/PEO/TMC | 14,000 | 29 | 260.8 ± 10.3[3,6] | 20 min. |
| 11 | Gly/PEO/TMC | 20,000 | 10 | 61.0 ± 0.5[1,2] | 4 |
| 12 | Gly/PEO/TMC | 20,000 | 20 | 169.0 ± 0.8[1,2] | 4 |
| 13 | Gly/PEO/TMC | 20,000 | 30 | 289.2 ± 5.6[1,2] | 4 |

1 = fiber (dimensions = 10 mm x 1.5 mm diameter)
2 = as polymerized
3 = reprecipitated
4 = not determined
5 = not at equilibrium by day 13
6 = film (dimensions = 12 x 4 x 0.6 mm)

Several generalizations about the data in Table IV can be made. The time to reach an equilibrium value of water uptake depends on the shape of the sample (Example 7 fiber vs. film). It would also appear that the time to reach an equilibrium value of water uptake decreases as the PEO content increases.

Within the scatter in the data, equilibrium water uptake is linearly related to the PEO content in the range 5-30%. There is no noticeable effect of the MW of the PEO block on the swelling of these triblock polymers (within the range of PEO MW 8,000-20,000).

One important difference noted in Table IV is the contrast of Example 10 (Gly/PEO/TMC) with Example 18 (Gly/PEO/dl-Lactide). Both have approximately the same percent of PEO 8,000; however, a reprecipitated sample of Example 10 had an equilibrium water content of 124% (Teg 1 day) vs. 9.9% by day 13 for a reprecipitated sample of Example 18. The difference can be rationalized by looking at the differences of the two matrices. In the case of the sample of Example 10 the rubbery Gly/TMC matrix is free to deform to accommodate the dimensional changes caused by the swelling. In Example 18 however the Gly/dl-Lactide matrix is in a glassy state. This should result in a slower water uptake curve (note that at 13 days equilibrium has not been reached) until the Gly/dl-Lactide matrix is sufficiently plasticized by water.

Examples 33-44

In Vitro Release of bST at 37°C, pH = 7.4

In vitro release of bST was measured for a number of hydrogel compositions (Table VII) and fabrication methods. The results indicate that, in general, bST release rates increase as the PEO content of the hydrogel increases. As previously discussed, higher PEO content leads to increased equilibrium water uptake which should allow for faster bST diffusion through the swollen gel. Several other trends are apparent from the results in Table VII. The fabrication method greatly influences the release rates of bST from the hydrogels. In general, it was found that extruded fibers gave lower release rates than solution cast films. The cast films contained a large number of voids and often delaminated due to the drying process. This gave a formulation with a much higher surface to volume ratio as compared to the extruded fibers. This high surface/volume ratio accounts for the high release after only 1 day for the solution cast films.

No discernible differences in release rates as a function of PEO mw could be detected. Again, this is expected as it previously has been shown that, in the mw range studied, PEG mw did not influence the swelling behavior of the hydrogels. Finally, it has been demonstrated that release rates can be modified by blending various additives into the formulations. A blend of bST/hydrogel (Example 10) and a Gly/L-lactide (40/60) polymer $\eta$inh = 0.50 (25/50/25) was extruded. The measured in vitro release rate of the blend was approximately 2/3 of the release rate for the hydrogel. By blending in a non-swelling Gly/L-lactide polymer, it serves to lower the overall PEO content and reduce the water uptake of the hydrogel. The measured release rates can also be increased by blending in a water soluble filler. When sorbitol was added to the previously described blend, the measured release rates were greater than the parent hydrogel release rates. The water soluble filler is leached out into the dissolution media leaving a more porous matrix which facilitates the release of the active material.

## TABLE VII

### In Vitro Release of bST
### $37^{o}C$, pH 7.4

| Example No. | Polymer Example | PEG MW | PEG Content (wt %) | bST Loading | Fabrication Method[C] | Day | | | | | |
|---|---|---|---|---|---|---|---|---|---|---|---|
| | | | | | | 1 | 2 | 3 | 4 | 5 | 6 |
| 33 | 10 | 8,000 | 8 | 25 | SC | 76.9 | 78.5 | ---- | ---- | ---- | 79.1 |
| 34 | 10 | 8,000 | 8 | 25 | EX | 22.1 | 30.2 | ---- | ---- | 31.2 | 32.7 |
| 35 | 10 | 8,000 | 8 | 25[A] | EX | 14.4 | 17.1 | ---- | ---- | 20.2 | 20.2 |
| 36 | 10 | 8,000 | 8 | 25[B] | EX | 38.0 | 41.9 | ---- | ---- | ---- | 36.9 |
| 37 | 9 | 8,000 | 21 | 25 | SC | 75.6 | 78.8 | ---- | ---- | ---- | 77.8 |
| 38 | 8 | 8,000 | 31 | 25 | SC | 76.0 | 88.2 | 77.4 | ---- | ---- | 89.2 |
| 39 | 6 | 14,000 | 11 | 25 | SC | 60.2 | 67.4 | 66.4 | -------- | | 77.9 |
| 40 | 5 | 14,000 | 19 | 25 | SC | 75.0 | 76.6 | ---- | ---- | ---- | 79.0 |
| 41 | 7 | 14,000 | 29 | 25 | SC | 84.0 | 84.7 | ---- | ---- | ---- | 86.7 |
| 42 | 11 | 20,000 | 10 | 25 | EX | ---- | 37.6 | ---- | ---- | 58.6 | ---- |
| 43 | 12 | 20,000 | 20 | 25 | EX | 61.9 | 78.9 | ---- | ---- | 78.8 | ---- |
| 44 | 13 | 20,000 | 30 | 25 | EX | 39.6 | 61.2 | ---- | ---- | 85.5 | ---- |

A = Formulation is a blend (25% bST/50% Ex. 10/25% Gly/L-lact $\eta$inh=0.50
B = Formulation is a blend (25% bST/25% Ex. 10/25% sorbitol/25% Gly/L-lact $\eta$inh=0.50
C = EX = extruded
    SC = solution cast

EP 0 258 780 B1

Examples 47-48

In Vivo Release of bST in Hypox Rats

Based on the in vitro release curves, two formulations were tested for in vivo release of bST in hypox rats. The experimental details of the in vivo measurements were discussed previously. The results are shown in Table VIII. Both formulations show growth in hypox rats throughout the 10 day test period.

TABLE VIII

| In Vivo Release Data 25% bST Loaded Ground Matrices | | | | | |
|---|---|---|---|---|---|
| Ex. No. | % PEO | PEO MW | Weight Gain (grams) | | |
| | | | 0-3 Days | 0-7 Days | 0-10 Days |
| 47 | 10 | 8,000 | 5.6 ± 1.0 | 10.0 ± 1.4 | 12.3 ± 1.5 |
| 48 | A | A | 8.8 ± 0.86 | 13.8 ± 1.9 | 15.6 ± 2.0 |
| | Control[B] | | 8.6 ± 1.3 | 15.8 ± 1.3 | 25.5 ± 1.7 |
| A = Blend 33% [Example 10] 67% [(Gly/lact) 40/60 $\eta$inh = 0.50] B = 10 injections (80 $\mu$g/day) | | | | | |

**Claims**
**Claims for the following Contracting States : BE, CH, DE, FR, GB, IT, LI, LU, NL, SE**

1. A slow release drug delivery system comprising
   a drug being a biologically active material having a molecular weight of > 1000 and
   an ABA block polymer, wherein the (B) block is a poly(ethylene oxide) and the blocks (A) are comprised of degradable random copolymers of (1) glycolide and (2) trimethylene carbonate.

2. A drug delivery system according to Claim 1, wherein the drug is bovine somatotropin.

**Claims for the following Contracting States : AT, ES, GR**

1. A process for the preparation of a slow release drug delivery system which comprises admixing a drug being a biologically active material having a molecular weight of > 1000 and
   an ABA block polymer, wherein the (B) block is a poly(ethylene oxide) and the blocks (A) are comprised of degradable random copolymers of (1) glycolide and (2) trimethylene carbonate, and transforming the mixture thus obtained into a form suitable for administration.

2. The process according to Claim 1, wherein the drug is bovine somatotropin.

**Patentansprüche**
**Patentansprüche für folgende Vertragssaaten : BE, CH, DE, FR, GB, IT, LI, LU, NL, SE**

1. Ein System zur langsamen Abgabe von Arzneimittel, umfassend ein Arzneimittel, das ein biologisch aktives Material ist und ein Molekulargewicht von > 1000 hat und ein ABA-Blockpolymer, worin der (B)-Block ein Poly(ethylenoxid) ist und die Blöcke (A) aus abbaubaren statistischen Copolymeren von (1) Glykolid und (2) Trimethylencarbonat zusammengesetzt sind.

2. Ein Arzneimittel-Abgabesystem nach Anspruch 1, worin das Arzneimittel Rindersomatotropin ist.

EP 0 258 780 B1

**Patentansprüche für folgende Vertragsstaaten : AT, ES, GR**

1. Ein Verfahren zum Herstellen eines Systems zur langsamen Abgabe von Arzneimittel, umfassend das Vermischen eines Arzneimittel, das ein biologisch aktives Material ist und ein Molekulargewicht von > 1000 hat und eines ABA-Blockpolymers, worin der (B)-Block ein Poly(ethylenoxid) ist und die Blöcke (A) aus abbaubaren statistischen Copolymeren von (1) Glykolid und (2) Trimethylencarbonat zusammengesetzt sind und Umwandeln der so erhaltenen Mischung in eine zur Verabreichung geeigneten Form.

2. Das Verfahren nach Anspruch 1, worin das Arzneimittel Rindersomatotropin ist.

**Revendications**
**Revendications pour les Etats contractants suivants : BE, CH, DE, FR, GB, IT, LI, LU, NL, SE**

1. Système de délivrance de médicament à libération lente, comprenant un médicament consistant en une substance biologiquement active ayant un poids moléculaire supérieur à 1 000, et un polymère séquencé ABA, la séquence (B) étant un polyoxyde d'éthylène, et les séquences (A) comprenant des copolymères statistiques dégradables (1) d'un glycolide et (2) de carbonate de triméthylène.

2. Système de délivrance de médicament selon la revendication 1, dans lequel le médicament est la somatotropine bovine.

**Revendications pour les Etats contractants suivants : AT, ES, GR**

1. Procédé de préparation d'un système de délivrance de médicament à libération lente comprenant le mélange d'un médicament consistant en une substance biologiquement active ayant un poids moléculaire supérieur à 1000 et un polymère séquencé ABA dans lequel la séquence (B) est un polyoxyde d'éthylène et les séquences (A) comprennent des copolymères statistiques dégradables (1) d'un glycolide et (2) de carbonate de triméthyléne, et la transformation du mélange ainsi obtenu en une forme appropriée pour une administration.

2. Procédé selon la revendication 1, dans lequel le médicament est la somatotropine bovine.

16